**Europäisches Patentamt**

**European Patent Office**

**Office européen des brevets**

(19)

(11) Veröffentlichungsnummer: **0 237 744**
**B1**

(12) **EUROPÄISCHE PATENTSCHRIFT**

(45) Veröffentlichungstag der Patentschrift:
**10.01.90**

(51) Int. Cl.⁴: **C07D 277/72**

(21) Anmeldenummer: **87101447.8**

(22) Anmeldetag: **03.02.87**

(54) **Verfahren zur Aufarbeitung von 2-Merkaptobenzthiazol enthaltenden Teeren.**

(30) Priorität: **14.02.86 DE 3604705**

(43) Veröffentlichungstag der Anmeldung:
**23.09.87 Patentblatt 87/39**

(45) Bekanntmachung des Hinweises auf die Patenterteilung:
**10.01.90 Patentblatt 90/2**

(84) Benannte Vertragsstaaten:
**BE DE FR GB IT NL**

(56) Entgegenhaltungen:
**US-A- 3 031 073**

**Die Akte enthält technische Angaben, die nach dem Eingang der Anmeldung eingereicht wurden und die nicht in dieser Patentschrift enthalten sind.**

(73) Patentinhaber: **BAYER ANTWERPEN N.V., Kanaaldok B 1 Kruisschans, B-2040 Antwerpen 4(BE)**

(72) Erfinder: **Denecker, Gabriel Dr., Vinkenlaan 18, B-2180 Kalmthout(BE)**
Erfinder: **Lahousse, Guido, Dr., Nerviersiei 11, B-2120 Schoten(BE)**
Erfinder: **Vandebroek, Henri, Dr., Nijverheidslaan 138 C, B-9170 Waasmunster(BE)**
Erfinder: **Bamelis, Pol, Dr., Im Holz 20, D-5060 Bergisch-Gladbach 1(DE)**

(74) Vertreter: **Gremm, Joachim, Dr. et al, Bayer AG Konzernverwaltung RP Patente Konzern, D-5090 Leverkusen 1, Bayerwerk(DE)**

## Beschreibung

Die Erfindung betrifft die Aufarbeitung von 2-Merkaptobenzthiazol enthaltenden teerartigen Rückständen, die nach der Herstellung des Produktes durch Umsetzung bei erhöhter Temperatur und erhöhtem Druck von Anilin, Schwefelkohlenstoff und Schwefel oder von Benzthiazol und Schwefel oder von Anilin, Schwefelkohlenstoff, Benzthiazol und Schwefel und anschließender extraktiver Reinigung des rohen Umsetzungsproduktes zurückbleiben.

Die technische Synthese von 2-Merkaptobenzthiazol durch Umsetzung von Anilin, Schwefelkohlenstoff und Schwefel oder von Benzthiazol und Schwefel oder von Anilin, Schwefelkohlenstoff, Benzthiazol und Schwefel bei Temperaturen im Bereich von etwa 220°C bis etwa 320°C und bei Drücken bis zu etwa 13 MPa ist bekannt.

Zur Reinigung wird das rohe 2-Merkaptobenzthiazol, nach Entspannung und Abtrennung von den niedrigsiedenden Bestandteilen, üblicherweise in einer wäßrigen anorganischen Base aufgenommen, anschließend von den nichtumgesetzten höhersiedenden Ausgangsmaterialien sowie von den diversen Zwischen- und Nebenkomponenten der Synthesereaktion durch Extraktion der wäßrigen 2-Merkaptobenzthiazolsalzlösung mit organischen wasserunlöslichen Extraktionsmitteln, wie Benzol, Toluol, Xylol oder Chlorkohlenwasserstoffen abgetrennt und durch Ansäuern isoliert (DE-PS 19 41 379) und DE-OS 2 709 989).

Nach DE-OS 2 652 394 und EP-OS 464 wird das rohe Umsetzungsprodukt der Synthesereaktion, ohne Einsatz von Säure durch Ausrühren in einem flüssigen Medium, beispielsweise Schwefelkohlenstoff oder Chlorkohlenwasserstoffen, in dem 2-Merkaptobenzthiazol praktisch unlöslich ist, direkt gefällt. Die Ausgangsmaterialien und die diversen Zwischen- und Nebenkomponenten der Synthesereaktion bleiben in Lösung.

Es ist bekannt, daß beim Aufdestillieren der Extraktphasen, welche bei den vorbeschriebenen Reinigungsverfahren anfallen, teerartige Rückstände verbleiben. Diese enthalten Anilin- und Benzthiazolfraktionen, welche im allgemeinen durch Vakuumdestillation abgetrennt und erneut als Ausgangsmaterial für die Synthesereaktion verwendet werden.

Weiter enthalten diese Teere nicht vernachlässigbare Mengen an 2-Merkaptobenzthiazol. Es ist vielfach versucht worden, diese 2-Merkaptobenzthiazolanteile zu nutzen.

So wird nach US-PS 3 031 073 eine vollständige Rückführung der Teere in die Synthesereaktion vorgeschlagen.

Dieses Verfahren hat jedoch den Nachteil, daß ein verfärbtes, Teerkomponenten enthaltendes 2-Merkaptobenzthiazol resultiert, das nicht als solches in der Vulkanisation von Kautschukmischungen oder als Ausgangsmaterial für die Synthese von anderen Thiazol- oder Sulfenamidbeschleunigern verwendet werden kann und wofür bisher kein ökonomisch arbeitendes Reinigungsverfahren bekannt ist.

Es wurde nun gefunden, daß man den Teerartigen Rückstand verwerten kann, indem man ihn einer thermischen Behandlung unterwirft und die dabei gebildeten Produkte Anilin und Benzthiazol in reiner Form abtrennt, insbesondere durch Vakuumdestillation. Diese Produkte können in üblicher Weise für die Herstellung von 2-Merkaptobenzthiazol verwendet werden.

Gegenstand der vorliegenden Erfindung ist somit ein Verfahren zur Aufarbeitung von 2-Merkaptobenzthiazol enthaltenden teerartigen Rückständen, das dadurch gekennzeichnet ist, daß man den Rückstand wenigstens eine Stunde auf Temperaturen oberhalb von 200°C erhitzt, und Anilin und Benzthiazol aus dem Produktgemisch isoliert.

Vorzugsweise wird der Rückstand 2 bis 15 Stunden auf 240°C bis 310°C erhitzt. Der Druck stellt sich hierbei spontan auf etwa 0.3 bis 3 MPa ein.

In einer weiteren Ausführungsform des erfindungsgemäßen Verfahrens wird die thermische Behandlung nach oder unter Aufdrücken von Wasserstoff und/oder Schwefelwasserstoff (etwa 0,3 bis 10 MPa) durchgeführt.

Für eine maximale Gewinnung von Anilin und Benzthiazol wird vorzugsweise das vor der thermischen Behandlung meistens vorhandene Anilin und Benzthiazol wenigstens teilweise aus dem aufzuarbeitenden Rückstand abdestilliert.

Die Gesamtausbeute an Anilin und Benzthiazol des Umwandlungs- und Spaltprozesses beträgt im allgemeinen 10 bis etwa 40 Gew.-%, bezogen auf eingesetzten Teer. Bei niedrigeren Temperaturen und/oder längeren Verweilzeiten werden kleinere Ausbeuten erhalten. Höhere Temperaturen führen verstärkt zu Verkrackungsreaktionen und zur Bildung von unerwünschten Komponenten, welche durch Destillation kaum von Benzthiazol zu trennen sind, z.B. 2-Methylbenzthiazol.

Das erfindungsgemäße Verfahren kann sowohl diskontinuierlich als auch kontinuierlich in allen für Druckreaktionen geeigneten Apparaten, beispielsweise Rührreaktoren, Mehrkammerreaktoren und Rohrreaktoren, durchgeführt werden.

Das erhaltene Produktgemisch wird anschließend im Vakuum fraktioniert. Als Kopfprodukt werden hierbei Destillate mit einem Gesamtgehalt an Anilin und Benzthiazol von mindestens 98 Gew.-% erhalten. Als Sumpfprodukt verbleibt ein teerartiger Rückstand, der normalerweise weniger viskos ist als vor der thermischen Behandlung und problemlos, z.B. in einem Clausofen zur Rückgewinnung des Schwefels und zur Verwertung des Energieinhaltes, verbrannt werden kann.

Die anilin- und benzthiazolhaltigen Destillate können mit Schwefelkohlenstoff und Schwefel, gegebenenfalls zusammen mit frischem Anilin und/oder Benzthiazol, in bekannter Weise zu 2-Merkaptobenzothiazol von hoher Reinheit umgesetzt werden.

2-Merkaptobenzthiazol wird als Vulkanisationsbeschleuniger für Kautschukmischungen und als Ausgangsmaterial für Benzthiazolsulfenamide, Benzthiazolyldisulfid (MBTS) und Merkaptobenzthiazolzinksalz (ZMBT) verwendet.

<u>Beispiel 1</u>

1000 g eines teerartigen Rückstandes aus der extraktiven Reinigung von rohem 2-Mercaptobenzthiazol (MBT) gemäß DE-PS 19 41 379 mit folgenden Bestandteilen:
Anilin : 0,3 Gew.-%
Benzthiazol : 4,8 Gew.-%
MBT : 14,5 Gew.-%
wurden 6 bis 7 Stunden unter Eigendruck auf 200 bis 315°C erhitzt. Nach Abkühlen wurden die nachfolgenden Analysenwerte und Nettoausbeuten ermittelt:

| Temperatur | (°C) | 200 | 250 | 275 | 300 | 315 |
|---|---|---|---|---|---|---|
| Reaktionszeit | (Std) | 7 | 6 | 7 | 7 | 7 |
| <u>Analyse:</u> | | | | | | |
| Anilin | (%) | 0,7 | 4,2 | 7,2 | 9,8 | 10,6 |
| Benzthiazol | (%) | 4,5 | 8,6 | 16,0 | 16,0 | 13,0 |
| 2-Methylbenzthiazol | (%) | 0,6 | 0,1 | 0,1 | 0,7 | 1,5 |
| Ausbeute* | (%) | 0,1 | 7,7 | 18,1 | 20,7 | 18,5 |

\* Netto-Ausbeute an Anilin und Benzthiazol bezogen auf eingesetzten Teer

<u>Beispiel 2</u>

1000 g eines teerartigen Rückstandes mit folgenden Bestandteile:
Anilin : 0,8 Gew.-%
Benzthiazol : 3,9 Gew.-%
MBT : 14,8 Gew.-%
wurden 7 Stunden auf 285°C erhitzt, einerseits unter Eigendruck, andererseits nach Aufdrücken von einer derartigen Menge Wasserstoff bzw. Schwefelwasserstoff, daß sich während der thermischen Behandlung ein Druck von 1,6-2,1 MPa einstellte.

Hierbei wurden Produktgemische enthalten, welche die nachfolgenden Ergebnisse aufweisen:

| | | Eigendruck | $H_2$-Atmosphäre | $H_2S$-Atmosphäre |
|---|---|---|---|---|
| <u>Analyse:</u> | | | | |
| Anilin | (%) | 6,6 | 11,0 | 9,2 |
| Benzthiazol | (%) | 9,0 | 12,0 | 8,9 |
| Ausbeute* | (%) | 10,9 | 18,3 | 13,4 |

\*Netto Ausbeute

<u>Beispiel 3</u>

1000 g teerartigen Rückstandes I wurden nach dem erfindungsgemäßen Verfahren thermisch behandelt und das dabei erhaltene Produktgemisch II anschließend bei 250°C/60 Torr (entspr. 79 hPa) fraktioniert. Es resultierten einerseits etwa 780 g eines verhältnismäßig wenig viskosen Sumpfproduktes III und andererseits 213 g eines anilin- und benzthiazolreichen Kopfproduktes IV. Die Gesamtausbeute an Anilin und Benzthiazol (bezogen auf dem eingesetzten Teer I) beträgt entsprechend den nachfolgenden Analysenwerte etwa 21 %:

| | | I | II | III | IV |
|---|---|---|---|---|---|
| Analyse: | | | | | |
| Anilin | (%) | 0,7 | 9,1 | 0,1 | 45,1 |
| Benzthiazol | (%) | 4,8 | 13,0 | 1,6 | 53,0 |
| 2-Methylbenzthiazol | (%) | | 0,3 | 0,1 | 0,4 |
| MBT | (%) | 12,0 | | | |

**Patentansprüche**

1. Verfahren zur Herstellung von 2-Mercaptobenzthiazol durch
a) Reaktion der Ausgangsmaterialien
1) Anilin, Schwefelkohlenstoff und Schwefel,
2) Benzthiazol und Schwefel oder
3) Anilin, Schwefelkohlenstoff, Benzthiazol und Schwefel
zu rohem 2-Mercaptobenzthiazol,
b) Lösen des rohen 2-Mercaptobenzthiazols mit einer wäßrigen organischen Base und extraktive Reinigung der erhaltenen Lösung mit einem organischen wasserunlöslichen Extraktionsmittel,
c) Gewinnung des 2-Marcaptobenzthiazols aus der wäßrigen Phase aus b),
d) Entfernung des Extraktionsmittels aus dem Extrakt aus b), wobei ein teeriger 2-Mercaptobenzthiazol enthaltender Rückstand verbleibt,
e) Aufarbeitung des Rückstands durch Erhitzen auf eine Temperatur von 240 bis 310°C unter einem autogenen Druck von 0,3 bis 3 MPa oder — nach oder unter Aufdrücken von Wasserstoff und/oder Schwefelwasserstoff — unter einem Druck von 0,3 bis 10 MPa für eine Zeitdauer von 2 bis 15 Stunden,
f) Abtrennung von Anilin und Benzthiazol — ggf. durch Vakuumdestillation — aus dem Reaktionsgemisch e).
2. Verfahren nach Anspruch 1, dadurch gekennzeichnet, daß der Rückstand vor der thermischen Behandlung durch Vakuumdestillation von Anilin und Benzthiazol wenigstens teilweise befreit wird.

**Claims**

1. Process for the preparation of 2-mercaptobenzothiazole by
a) reacting the starting materials
1) aniline, carbon disulphide and sulphur,
2) benzothiazole and sulphur or
3) aniline, carbon disulphide, benzothiazole and sulphur to give crude 2-mercaptobenzothiazole,
b) dissolving the crude 2-mercaptobenzothiazole using — aqueous organic base and extractively purifying the resulting solution using an organic water-insoluble extracting agent,
c) recovering the 2-mercaptobenzothiazole from the aqueous phase of b),
d) removing the extracting agent from the extract of b), leaving a tarry residue containing 2-mercaptobenzothiazole,
e) working up the residue by heating to a temperature of 240 to 310°C under an autogenous pressure of 0.3 to 3 MPa or — after or during forcing in of hydrogen and/or hydrogen sulphide — under a pressure of 0.3 to 10 MPa for a period of 2 to 15 hours,
f) separating off aniline and benzothiazole — if appropriate by vacuum distillation — from the reaction mixture e).
2. Process according to claim 1, characterized in that the residue is at least partly freed from aniline and benzothiazole by vacuum distillation before the heat treatment.

**Revendications**

1. Procédé pour la fabrication de 2-mercaptobenzothiazole par:
a) réaction des matériaux de départ
1) aniline, sulfure de carbone et soufre,
2) benzothiazole et soufre ou
3) aniline, sulfure de carbone, benzothiazole et soufre
pour obtenir du 2-mercaptobenzothiazole brut,
b) mise en solution du 2-mercaptobenzothiazole avec une base organique aqueuse et épuration de la solution obtenue par extraction avec un agent d'extraction organique non soluble dans l'eau,

EP 0 237 744 B1

c) obtention du 2-mercaptobenzothiazole à partir de la phase aqueuse de b),

d) élimination de l'agent d'extraction de l'extrait b), après quoi il reste un résidu contenant du 2-mercaptobenzothiazole,

e) traitement du résidu en le chauffant à une température de 240 à 310°C sous une pression autogène de 0,3 à 3 MPa ou – après ou sous mise en pression d'hydrogène et/ou de sulfure d'hydrogène – sous une pression de 0,3 à 10 MPa durant 2 à 15 heures,

f) séparation de l'aniline et du benzothiazole du mélange réactionnel e) – éventuellement par distillation sous vide.

2. Procédé selon la revendication 1, caractérisé en ce que le résidu est, avant le traitement thermique, débarrassé au moins partiellement de l'aniline et du benzothiazole par distillation sous vide.

5